# EUROPÄISCHE PATENTSCHRIFT

(11) **EP 0 639 169 B1**
(45) Veröffentlichungstag und Bekanntmachung des Hinweises auf die Patenterteilung: **08.01.1997**
(21) Anmeldenummer: 93911495.5
(22) Anmeldetag: 22.04.1993
(51) Int. Cl.: C07C 17/26, C07C 21/18

(54) **VERFAHREN ZUR HERSTELLUNG VON HEXAFLUORCHLORBUTENEN**
PROCESS FOR PRODUCING HEXAFLUOROCHLOROBUTENES
PROCEDE DE FABRICATION D'HEXAFLUOROCHLOROBUTENES

(30) Priorität: 04.05.1992 DE 4214739
(43) Veröffentlichungstag der Anmeldung: 22.02.1995
(73) Patentinhaber: BAYER AG, 51368 Leverkusen (DE)
(72) Erfinder: LUI, Norbert, D-5000 Koeln 80 (DE); MARHOLD, Albrecht, D-5090 Leverkusen (DE); BIELEFELDT, Dietmar, D-4030 Ratingen 6 (DE)
(86) Internationale Anmeldenummer: EP9300980
(87) Internationale Veröffentlichungsnummer: WO9322263

(56) Entgegenhaltungen:
- EP-A- 0 287 219
- US-A- 2 413 695
- JOURNAL OF FLUORINE CHEMISTRY Bd. 35, Nr. 1, Februar 1987, LAUSANNE CH Seite 204 MATAE IWASAKI ET AL. 'Decomposition of Some Hydrogen-Bearing Halogenated Ethanes'

## Beschreibung

Die vorliegende Erfindung betrifft ein Verfahren zur Herstellung von Hexafluorchlorbutenen aus Trifluordichlorethan.

Hexafluorchlorbutene sind bekannte Zwischenprodukte, aus denen sich Hexafluorbutane herstellen lassen, die neuerdings z.B. bei der Herstellung von Schaumstoffen als Ersatz für Fluorkohlenwasserstoffe (FCKW) von Interesse sind.

Aus der US-PS 2 413 695 ist bekannt, daß die Pyrolyse von 1,1,1-Trifluor-2-chlorethan, einer Monochlorverbindung, zu einer gesättigten C₄-Verbindung (C₄H₃ClF₆) führt.

Journal of Fluorine Chemistry, 1987, 35(1), 204 beschreibt ein Verfahren, wobei CF₃CHBrCl auf 470°C erhitzt wird und zu u.a. CF₃CH=CClCF₃ und CF₃CCl=CClCF₃ führt.

Es wurde nun ein Verfahren zur Herstellung von 1,1,1,4,4,4-Hexafluor-chlorbutenen gefunden, das dadurch gekennzeichnet ist, daß man 1,1,1-Trifluor-2,2-dichlorethan bei einer Temperatur im Bereich von 500 bis 700°C pyrolysiert.

Das für das erfindungsgemäße Verfahren als Ausgangsmaterial benötigte 1,1,1-Trifluor-2,2-dichlorethan wird in technischem Maßstab hergestellt und ist im Handel erhältlich. Es kann gegebenenfalls zusammen mit 1,1,1-Trifluor-2,2,2-trichlorethan eingesetzt werden.

Die erfindungsgemäße Pyrolyse wird bei Temperaturen im Bereich von 500 bis 700°C, insbesondere von 550 bis 650°C, durchgeführt.

Der Druck ist für das erfindungsgemäße Verfahren ohne besondere Bedeutung, solange sichergestellt ist, daß sich bei der gewählten Pyrolysetemperatur das Ausgangsmaterial in gasförmigem Zustand befindet. Der Druck kann z.B. 0,1 bis 50 bar betragen. Vorzugsweise arbeitet man bei Normaldruck.

Man kann die Pyrolyse gegebenenfalls in Gegenwart von inerten Gasen durchführen, z.B. in Gegenwart von Edelgasen oder Stickstoff. Bevorzugt ist es jedoch das 1,1,1-Trifluor-2,2-dichlorethan ohne irgendwelche Zusätze auf die Pyrolysetemperatur zu erhitzen.

Die Pyrolyse kann man z.B. so durchführen, daß man 1,1,1-Trifluor-2,2-dichlorethan einem oder mehreren parallel angeordneten Rohren aus inertem Material zuführt und das Rohr bzw. die Rohre auf die gewünschte Pyrolysetemperatur erhitzt. Geeignete Rohrmaterialien sind z.B. Quarz, Nickel und Nickel-, Chrom- und Molybdänstähle. Die Rohre können z.B. innere Durchmesser von 10 bis 50 mm aufweisen. Die Länge des Rohres bzw. der Rohre und die Strömungsgeschwindigkeit der Gase kann man beispielsweise so aufeinander abstimmen, daß sich Verweilzeiten im Bereich der Pyrolysetemperatur von 0,1 bis 120 Sekunden ergeben. Vorzugsweise beträgt die Verweilzeit von 1 bis 30 Sekunden.

Das Rohr bzw. die Rohre konnen gegebenenfalls mit inerten stückigen Materialien gefüllt sein, z.B. mit regelmäßig oder unregelmäßig geformten Quarzstücken mit einem mittleren Durchmesser von 1 mm bis zur Hälfte des inneren Durchmessers des jeweiligen Rohres. Solche inerten stückigen Materialien können den Wärmeübergang von der beheizten Rohrwand zum durchströmenden Gas verbessern.

Das aus der Hydrolysezone kommende Gasgemisch kann man beispielsweise aufarbeiten, indem man es ganz oder teilweise kondensiert und aus dem Kondensat durch Destillation die darin enthaltenen Hexafluorchlorbutene gemeinsam oder getrennt voneinander isoliert. Bei einer teilweisen Kondensation des aus der Pyrolyse kommenden Gasgemisches kann man z.B. die bei Normaldruck über -78°C, vorzugsweise die über 0°C kondensierbaren Anteile des Gasgemisches kondensieren.

Das hinter der Pyrolysezone vorliegende Gasgemisch enthält im allgemeinen die gewünschten Produkte 1,1,1,4,4,4-Hexafluor-2-chlorbuten-2 und 1,1,1,4,4,4-Hexafluor-2,3-dichlorbuten-2, die beispielsweise in einem Molverhältnis im Bereich von 35:65 bis 65:35 vorliegen können, sowie 1,1,1-Trifluor-2,2,2-trichlorethan, unumgesetztes Ausgangsprodukt, Chlor und gegebenenfalls weitere Komponenten in geringen Mengen.

Nichtumgesetztes 1,1,1-Trifluor-2,2-dichlorethan und 1,1,1-Trifluor-2,2,2-trichlorethan können erneut der erfindungsgemäßen Pyrolyse zugeführt werden. Chlor kann für die Herstellung des Ausgangsmaterials verwendet werden. Die erhaltenen Hexafluorchlorbutene kann man gemeinsam einer Hydrierung unterwerfen und so 1,1,1,4,4,4-Hexafluorbutan erhalten, das als FCKW-freies Treibgas für die Herstellung von Schaumstoffen verwendet werden kann.

Die Hydrierung von 1,1,1,4,4,4-Hexafluor-2-chlorbuten-2 und 1,1,1,4,4,4-Hexafluor-2,3-dichlorbuten-2 zu 1,1,1,4,4,4-Hexafluorbutan kann man beispielsweise katalytisch und in der Gasphase durchführen. Für diese Hydrierung geeignete Temperaturen liegen beispielsweise im Bereich von 80 bis 400°C, geeignete Katalysatoren sind beispielsweise Übergangsmetalle auf Trägermaterialien, insbesondere Palladium und Platin auf Aktivkohlen und Lithium-Aluminium-Spinellen. Geeignete Drucke für diese Hydrierung sind beispielsweise solche zwischen Normaldruck und 20 bar. Ein derartiges Hydrierverfahren ist beispielsweise in der DE-OS 40 04 497 beschrieben. Es kommen prinzipiell jedoch auch andere Hydrierverfahren in Frage.

Das erfindungsgemäße Verfahren benötigt zur Herstellung von 1,1,1,4,4,4-Hexafluor-chlorbutenen keinen Wasserstoff, keine Katalysatoren, geht von leicht zugänglichen Ausgangsmaterialien aus, liefert einfach abzutrennendes und gut wiederverwendbares Chlor als Nebenprodukt und ist auf einfache Weise durchführbar. Es ist ausgesprochen überraschend, daß im Gegensatz zu dem eingangs geschilderten Stand der Technik gemäß der vorliegenden Erfindung aus einer Dichlorethanverbindung durch Pyrolyse ungesättigte Hexafluor-chlorbutene erhältlich sind.

### Beispiele

### Beispiel 1

In einem Vorverdampfer bestehend aus einem 20 cm langen Quarzrohr (Innendurchmesser 25 mm) und gefüllt mit partikelförmigem Quarz wurden stündlich 60 g 1,1,1-Trifluor-2,2-dichlorethan verdampft und auf 100°C erwärmt. Anschließend wurde das so erhaltene Gas einem 35 cm langen Quarzrohr zugeführt (Innendurchmesser 25 mm) das ebenfalls mit partikelförmigem Quarz gefüllt war und elektrisch auf 625°C beheizt wurde. Die das zweite Quarzrohr verlassende Gase wurden bei 15°C kondensiert und anschließend gaschromatographisch untersucht. Im Laufe einer Stunde wurden 50 g eines Gemisches erhalten, das 40 Gew.-% Hexafluorbutene (etwa gleiche Gew.-Teile 1,1,1,4,4,4-Hexafluor-2-chlorbuten-2 und 1,1,1,4,4,4-Hexafluor-2,3-dichlorbuten-2), 31 Gew.-% nicht umgesetztes 1,1,1-Trifluor-2,2-dichlorethan und 17 Gew.-% 1,1,1-Trifluor-2,2,2-trichlorethan enthielt. Das eingesetzte 1,1,1-Trifluor-2,2-dichlorethan hatte sich zu 72 % umgesetzt, Hexafluorchlorbutene, die sich nach destillativer Abtrennung zu Hexafluorbutan hydrieren lassen, hatten sich mit einer Selektivität von 57 % gebildet.

### Beispiel 2

Die bei der Aufarbeitung gemäß Beispiel 1 erhaltenen Trifluorchlorethane (1,1,1-Trifluor-2,2-dichlorethan und 1,1,1-Trifluor-2,2,2-trichlorethan) wurden vereinigt und mit frischem 1,1,1-Trifluor-2,2-dichlorethan auf 60 g aufgefüllt. Die so erhaltene Mischung wurde wie in Beispiel 1 beschrieben pyrolysiert und das erhaltene Kondensat gaschromatographisch untersucht. Im Verlaufe 1 Stunde hatten sich 52 g eines Gemisches gebildet, das zu 37 Gew.-% aus Hexafluorchlorbutenen (46 Gew.-% 1,1,1,4,4,4-Hexafluor-2-chlorbuten-2 und 54 Gew.-% 1,1,1,4,4,4-Hexafluor-2,3-dichlorbuten-2) bestand.

## Patentansprüche

1. Verfahren zur Herstellung von 1,1,1,4,4,4-Hexafluor-chlorbutenen, dadurch gekennzeichnet, daß man 1,1,1,-Trifluor-2,2-dichlorethan bei einer Temperatur im Bereich von 500 bis 700°C pyrolysiert.

2. Verfahren nach Anspruch 1, dadurch gekennzeichnet, daß man 1,1,1-Trifluor-2,2-dichlorethan im Gemisch mit 1,1,1-Trifluor-2,2,2-trichlorethan eingesetzt.

3. Verfahren nach Ansprüchen 1 bis 2, dadurch gekennzeichnet, daß man es bei Drücken im Bereich 0,1 bis 50 bar durchführt.

4. Verfahren nach Ansprüchen 1 bis 3, dadurch gekennzeichnet, daß man es in Rohren aus inertem Material durchführt.

5. Verfahren nach Ansprüchen 1 bis 4, dadurch gekennzeichnet, daß man Verweilzeiten im Bereich der Pyrolysetemperatur von 0,1 bis 120 Sekunden einhält.

6. Verfahren nach Anspruch 4, dadurch gekennzeichnet, daß die Rohre mit inerten stückigen Materalien gefüllt sind.

7. Verfahren nach Ansprüchen 1 bis 6, dadurch gekennzeichnet, daß man das aus der Pyrolysezone kommende Gasgemisch kondensiert und aus dem Kondensat durch Destillation die darin enthaltenen Hexafluorchlorbutene isoliert.

## Claims

1. A process for preparing 1,1,1,4,4,4-hexafluorochlorobutenes, characterised in that 1,1,1-trifluoro-2,2-dichloroethane is pyrolysed at a temperature in the range 500 to 700°C.

2. A process according to Claim 1, characterised in that 1,1,1-trifluoro-2,2-dichloroethane mixed with 1,1,1-trifluoro-2,2,2-trichloroethane is used.

3. A process according to Claims 1 to 2, characterised in that it is performed at pressures in the range 0.1 to 50 bar.

4. A process according to Claims 1 to 3, characterised in that it is performed in tubes made of inert material.

5. A process according to Claims 1 to 4, characterised in that residence times of 0.1 to 120 seconds are maintained in the region of the pyrolysis temperature.

6. A process according to Claim 4, characterised in that the tubes are filled with inert particulate materials.

7. A process according to Claims 1 to 6, characterised in that the gas mixture emerging from the pyrolysis zone is condensed and the hexafluorochlorobutenes contained therein are isolated from the condensate by distillation.

## Revendications

1. Procédé de préparation de 1,1,1,4,4,4-hexafluoro-chlorobutènes, caractérisé en ce que l'on pyrolyse le 1,1,1-trifluoro-2,2-dichloréthane à des températures dans l'intervalle de 500 à 700°C.

2. Procédé selon la revendication 1, caractérisé en ce que le 1,1,1-trifluoro-2,2-dichloréthane est mis en oeuvre en mélange avec du 1,1,1-trifluoro-2,2,2-trichloréthane.

3. Procédé selon les revendications 1 et 2, caractérisé en ce que l'on opère sous des pressions qui se situent dans l'intervalle de 0,1 à 50 bar.

4. Procédé selon les revendications 1 à 3, caractérisé en ce que l'on opère dans des tubes en matériaux inertes.

5. Procédé selon les revendications 1 à 4, caractérisé en ce que l'on observe des durées de passage de 0,1 à 120 s dans l'intervalle des températures de pyrolyse.

6. Procédé selon la revendication 4, caractérisé en ce que les tubes sont garnis de matériaux inertes en morceaux.

7. Procédé selon les revendications 1 à 6, caractérisé en ce que l'on condense le mélange gazeux sortant de la zone de pyrolyse et on isole par distillation du condensat les hexafluorobutènes qu'il contient.
